Europäisches Patentamt

European Patent Office

Office européen des brevets

(19)

(11) Veröffentlichungsnummer: **0 004 888**
**B1**

(12) **EUROPÄISCHE PATENTSCHRIFT**

(45) Veröffentlichungstag der Patentschrift: **11.11.81**

(21) Anmeldenummer: **79100960.8**

(22) Anmeldetag: **29.03.79**

(51) Int. Cl.³: **A 61 B 5/10, A 61 C 19/04, G 01 B 7/00, G 01 R 33/06, G 01 D 5/14**

(54) **Vorrichtung zur Messung des Ortes, der Lage und/oder der Orts- bzw. Lageänderung eines starren Körpers im Raum.**

(30) Priorität: **04.04.78 DE 2814551**

(43) Veröffentlichungstag der Anmeldung:
**31.10.79 Patentblatt 79/22**

(45) Bekanntmachung des Hinweises auf die Patenterteilung:
**11.11.81 Patentblatt 81/45**

(84) Benannte Vertragsstaaten:
**FR GB IT**

(56) Entgegenhaltungen:
**FR - A - 2 386 807**
**US - A - 3 461 421**
**US - A - 3 822 694**
**IEE TRANSACTIONS ON INSTRUMENTATION AND MEASUREMENT, Vol. IM—26, Nr. 2, 1977—06 New York, U.S.A.**
**W.D. McCALL, Jr. et al.: "A Linear Position Transducer Using a Magnet and Hall Effect Devices", Seiten 133 bis 136.**

(73) Patentinhaber: **SIEMENS AKTIENGESELLSCHAFT**
**Berlin und München**
**Postfach 22 02 61**
**D-8000 München 22 (DE)**

(72) Erfinder: **Lewin, Arthur, Prof. Dr.**
**University of the Witwatersrand 1 Jan Smuts Av.**
**Johannesburg 2001 (ZA)**
Erfinder: **Nickel, Berndt, Dipl.-Ing.**
**Goldregenstrasse 2**
**D-6143 Lorsch (DE)**

**0 004 888**

## Vorrichtung zur Messung des Ortes, der Lage und/oder der Orts- bzw. Lageänderung eines starren Körpers im Raum

Die Erfindung bezieht sich auf eine Vorrichtung zur Messung und Registrierung des Ortes, der Lage und/oder der Orts- bzw. Lageänderung eines starren Körpers im Raum insbesondere einer S'telle des Unterkiefers eines Patienten unter Verwendung eines am Körper direkt oder im Abstand davon angeordneten Magnetfelderzeugers, mit im Abstand vom Felderzeuger angeordneten, vom Körper unabhängigen Mitteln zur Erfassung des Feldflusses bzw. der Feldflußänderung während einer Messung sowie mit einer elektronischen Einrichtung zur Gewinnung und Auswertung von bei einem Feldfluß bzw. einer Feldflußänderung entstehenden elektrischen Signalen.

Bei einer bekannten Vorrichtung dieser Art (US—A— 38 22 694) ist als Felderzeuger ein stabförmiger Dauermagnet mit N—Pol am einen und S—Pol am anderen Ende vorgesehen. Mit einem solchen Felderzeuger wird ein regelmäßiges, in bezug auf die Stabachse symmetrisches Feld erzeugt. Damit lassen sich aber insbesondere keine Rotationsbewegungen erfassen.

Um sowohl die Lage als auch Lageänderungen eines Körpers im Raum eindeutig erfassen zu können und letzteres nicht nur bei Translationsbewegungen, sondern auch bei Rotationsbewegungen des Körpers, wurde in der älteren deutschen Patentanmeldung P 27 15 106 vorgeschlagen, einen Felderzeuger vorzusehen, der ein definiertes, in bezug auf seine Rotationssymmetrieachse unregelmäßiges Feld erzeugt. Das Feld dieses Felderzeugers ist also bezüglich der geometrischen Achsen (x, y, z), deren gemeinsamer Mittelpunkt im geometrischen Zentrum des Felderzeugers liegt, nicht rotationssymmetrisch. Bei dem älteren Vorschlag sind zur Erfassung des Feldflusses mehrere senkrecht zueinander angeordnete Flächenpaare vorgesehen, wobei jedes Flächenpaar eine Vielzahl von Sensorelementen enthält.

Die Anordnung eines solchen Flächengebildes ist relativ voluminös und schwer und insbesondere bei Anwendung in der Zahnmedizin zur Erfassung der genauen Lage bzw. einer Lageänderung eines Punktes des Unterkiefers eines Patienten mit dem Nachteil verbunden, daß der direkte Zugang zum Mundbereich des Patienten von vorne behindert und dadurch die Zufuhr von Kaugut für eine Messung der Kaubewegung erschwert ist.

Es hat sich ferner gezeigt, daß die Signale, die Aufschluß für eine Rotationsbewegung geben, bei dieser Anordnung relativ klein und damit schlecht auswertbar sind, insbesondere wenn der Magnetfelderzeuger sich im Zentrum des Abnehmersystems befindet, weil hier ohnehin die Differenzsignale sehr klein sind. Die Bildung von weiteren Differenzsignalen aus den erhaltenen Differenzsignalen führt zu noch kleineren Signalen, die infolge des ohnehin vorhandenen und nicht zu vermeidenden Rauschens der Elektronikeinrichtung relativ schlecht und ungenau auswertbar sind.

Um den Einfluß des Erdmagnetfeldes zu kompensieren, ist bei dieser Anordnung außerdem eine Kompensationsschaltung für die drei Ebenen der Feldaufnahmer erforderlich, die einen zusätzlichen schaltungstechnischen Aufwand erfordert.

Aufgabe der Erfindung ist es, eine verbesserte Vorrichtung zu schaffen, die insbesondere vom Aufbau her einfacher ist, leichter und damit günstiger an einer Meßstelle zu haltern ist, die besser auswertbare Signale liefert und die einen verbesserten Zugang zum Bereich des Felderzeugers schafft. Ein weiteres Ziel der Erfindung ist, die Elektronik zur Gewinnung der Signale zu vereinfachen, insbesondere im Hinblick auf die Kabelund Leitungsführung.

Die gestellte Aufgabe wird bei einer Vorrichtung der eingangs genannten Art erfindungsgemäß dadurch gelöst, daß der Felderzeuger so ausgebildet ist, daß er ein definiertes, in bezug auf seine Rotationssymmetrieachse unregelmäßiges Feld erzeugt, daß zur Erfassung des Feldflusses in wenigstens zwei, vorzugsweise in drei, senkrecht zueinander stehenden Ebenen in jeder Erfassungsebene ein Paar jeweils aus einem Hallgenerator als Sensorelement und einem Antennenelement gebildete Magnetflußaufnehmer vorgesehen sind, die in einem Abstand voneinander auf einem Träger so angeordnet sind, daß zwischen den Magnetflußaufnehmern ein Freiraum gebildet ist.

Vorteilhafte Weiterbildungen und Ausgestaltungen der Erfindung sind in den Unteransprüchen angegeben.

Durch die Verwendung von Hallgeneratoren als Magnetflußaufnehmer zur Erfassung des Magnetflusses bzw. einer Magnetflußänderung in Verbindung mit Antennenelementen gelingt es, mit nur sechs Aufnehmern alle Bewegungen des Körpers, also sowohl die Translationsals auch die Rotationsbewegungen, aufzunehmen. Aufgrund der paarweisen Anordnung der Aufnehmer wird ein großflächiges und voluminöses Gebilde vermieden. Die gesamte Aufnehmeranordnung ist dadurch besser zu handhaben und auch gewichtsmäßig leichter. Ein besonderer Vorteil liegt in der Verwendung der Vorrichtung in der Zahnmedizin zur Erfassung der genauen Lage bzw. einer Lageänderung einer Stelle im Unterkiefer eines Patienten. Durch den Freiraum, der sich durch die auf Abstand angeordneten Magnetflußaufnehmer ergibt, wird eine direkter Zugang von vorne zum Magnetfelderzeuger erzielt und damit die Fixierung und Befestigung desselben sowie — bei Anwendung in der Zahnmedizin — das Zuführen von Kaugut an den Patienten erleichtert.

Ein weiterer wesentlicher Vorzug ist, daß kein zusätzlicher Aufwand für eine Kompensationsschaltung zur Vermeidung eines Einflusses der Erdmagnetfeldes notwendig ist. Durch die Anordnung einer Multiplexschaltungseinheit im Bereich der Magnetflußaufnehmeranordnung kann die Anzahl der Leitungen erheblich reduziert werden, so daß nurmehr ein relativ dünnes Verbindungskabel vom Aufnehmersystem zur Elektronic erforderlich ist.
erforderlich ist.

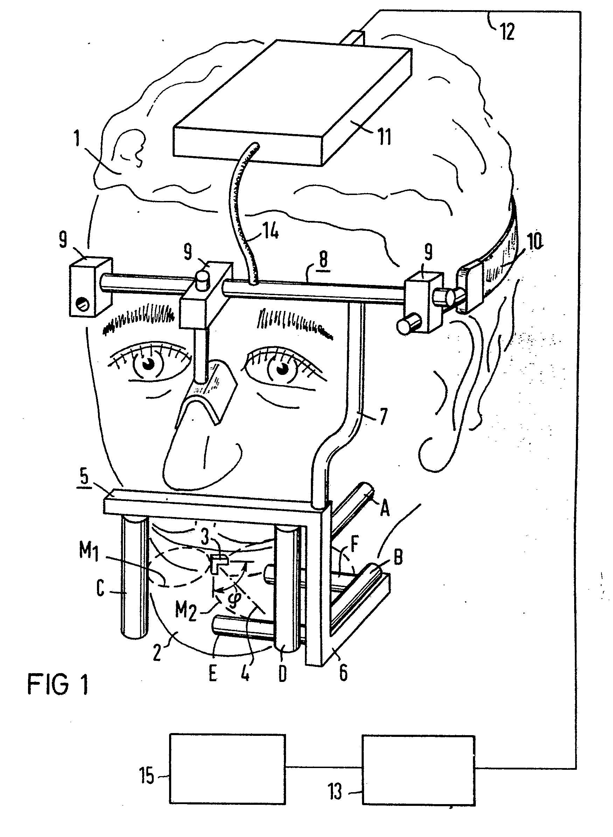

Ein Ausführungsbeispiel der Erfindung wird anhand der Figuren 1 bis 6 näher erläutert.

Die Fig. 1 zeigt in einer schaubildlichen und schematischen Darstellung die erfindungsgemäße Meßvorrichtung, angewendet in der Zahnmedizin zur Bestimmung des Ortes, der Lage und/oder einer Orts- bzw. Lageänderung eines Punktes des Unterkiefers eines Patienten. In der Figur ist mit 1 der Kopf und mit 2 der Unterkiefer eines Patienten bezeichnet. Mit 3 ist ein als Felderzeuger dienender Permanentmagnet bezeichnet, der intraoral an einer beliebigen Stelle des Unterkiefers durch geeignete Hafte- oder Klebemittel (z.B. Abdruckmasse) befestigt wird. Der Magnetfelderzeuger 3 besteht aus zwei gleichdimensionierten Stabmagneten, wie sie in der deutschen Patentanmeldung P 27 15 106 näher beschrieben sind, jedoch mit dem Unterschied, daß der Öffnungswinkel $\varphi$ etwa 90° beträgt. Die Stabmagnete des Magnetfelderzeugers 3 sind relativ klein; sie haben eine Länge von etwa 3 mm und einen Querschnitt von 1 mm im Quadrat. Die Winkelhalbierende der beiden Stabmagnete ist mit 4 bezeichnet. Der Magnetfelderzeuger 3 erzeugt zwei in der Figur gestrichelt angedeutete Magnetfelder $M_1$, $M_2$, und zwar im Berührungspunkt der gleichnamigen Pole (z.B. N—N) ein relativ kleines Feld $M_1$ und an den freien Schenkeln ein relativ großes Feld $M_2$.

Extraoral ist eine Magnetflußaufnehmeranordnung 5 angeordnet, die sechs stabförmige Magnetflußaufnehmer A bis F enthält, die auf einem Tragrahmen 6 befestigt sind. Der Tragrahmen 6 ist mit einer Tragstange 7 verbunden, die wiederum an einem als Brillen- oder Kopfgestell ausgebildeten Gestänge 8 befestigt ist. Das Gestänge 8 enthält mehrere Gelenke 9, die ein Anpassen und Einstellen der gesamten Einrichtung an den Patienten, an dem eine Messung vorgenommen werden soll, ermöglichen. Um das gesamte Gestänge fest am Patientenkopf 1 fixieren zu können, ist um den Kopf des Patienten ein Befestigungsband 10 gelegt.

Die Anordnung des Magnetfelderzeugers 3 und der Magnetflußaufnehmer A bis F ist so getroffen, daß die Winkelhalbierende 4 etwa den senkrechten Teil des Tragrahmens 6 schneidet. Der Magnetflußaufnehmer C ist außerdem so weit nach außen versetzt, daß das vom Magnetfelderzeuger erzeugte Magnetfeld $M_2$, welches von den freien Enden der beiden Stabmagnete ausgeht, nur von den Atennen A, B, D, E und F aufgenommen wird und das von den mit ihren gleichnamigen Polen aneinandergrenzenden Enden der Stabmagnete ausgehende wesentlich kleinere Magnetfeld $M_1$ im wesentlichen nur vom Magnetflußaufnehmer C aufgenommen wird.

Mit 11 ist eine später noch näher erläuterte, für eine Halterung auf dem Kopf des Patienten ausgebildete Multiplexeinrichtung bezeichnet, die einerseits über ein Verbindungskabel 12 mit einer Elektronikeinheit 13 und andererseits über ein Kabel 14 mit in den Magnetflußaufnehmern A bis F angeordneten Sensorelementen verbunden ist. Der Ausgang der Elektronikeinheit 13 ist mit einem an sich bekannten Indikator 15 verbunden, an dem die erfaßten und ausgewerteten Signale optisch angezeigt werden.

Der Magnetfelderzeuger 3 ist im Patientenmund so befestigt, daß der eine Schenkel etwa parallel zu einer durch die Aufnehmer E und F gebildeten Ebene, und der andere Schenkel etwa parallel zu einer durch die Aufnehmer C und D gebildeten Ebene verläuft, wobei die Öffnung des Magnetfelderzeugers (Winkelhalbierende 4) zu den Aufnehmern hin gerichtet ist.

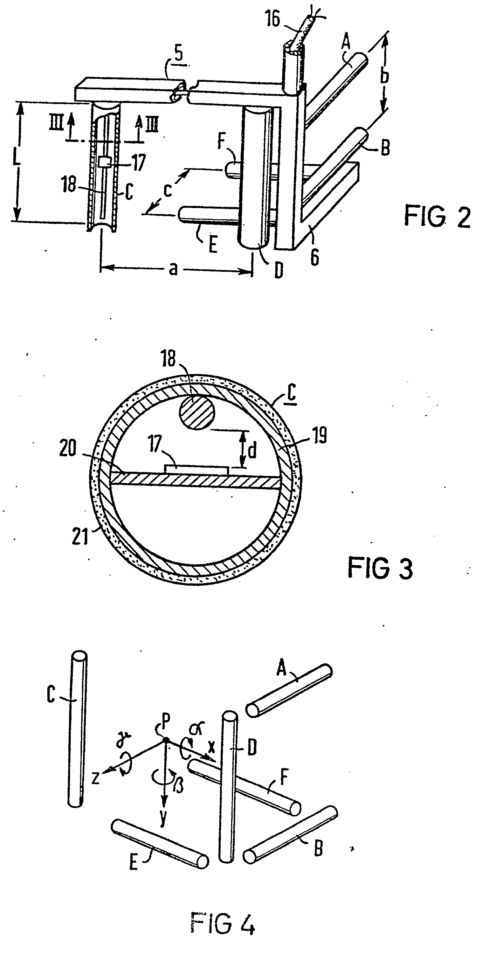

Die Fig. 2 zeigt die Magnetflußaufnehmeranordnung 5 mit den Magnetflußaufnehmern A bis F, wobei der Magnetflußaufnehmer C im Längsschnitt dargestellt ist. Aus der Darstellung ist ersichtlich, daß die Magnetflußaufnehmer jeweils paarweise- am Tragrahmen 6 gehaltert sind, wobei die Magnetflußaufnehmer eines jeden Paares in einer Ebene und in einem Abstand a, b, c, zueinander angeordnet sind. Der Rahmen 6 ist ⌐ förmig ausgebildet, wobei die beiden freien Schenkel des Rahmens abgewinkelt sind, also in zwei senkrecht zueinander stehenden Ebenen liegen, und besteht aus einem Hohlprofil, in dem Signal- und Versorgungsleitungen 16 von und zu den einzelnen Magnetflußaufnehmern A bis F führen. Die Magnetflußaufnehmer A bis F sind stabförmig ausgebildet und nehmen in ihrem Inneren je einen als Sensorelement dienenden Hallgenerator 17 sowie je einen Antennenstab 18 auf. Der Antennenstab 18 ist dem Magnetfelderzeuger zugewandt angeordnet und besteht jeweils aus Mu-Metall. Er erstreckt sich jeweils über die gesamte Länge eines Magnetflußaufnehmers. Der Hallgenerator 17 ist etwa auf halber Länge des Antennenstabes angeordnet. Die Abstände a, b, c und die jeweilige Länge L der Magnetfeldaufnehmer A bis F bzw. der Antennen 18 werden durch die Bewegungsbereiche des zu messenden Objekts (Unterkiefer) bestimmt.

Weitere Einzelheiten über den Aufbau der Magnetflußaufnehmer läßt die Fig. 3, die einen Querschnitt entlang der Linie III—III in Fig. 2 zeigt, erkennen.

Der Magnetflußaufnehmer C besteht, wie auch die übrigen Aufnahmer A, B und D bis F, im wesentlichen aus einem Rohr 19 von etwa 9 mm Außendurchmesser, einer im Rohr 19 angeordneten Trägerplatte 20 für den Hallgenerator 17, dem Hallgenerator 17 und dem am Rohr 19 befestigten Antennenstab 18. Um das Rohr 19 ist ein formelastischer Mantel 21, z.B. aus Schaumstoff, angeordnet. Das Rohr 19 besteht aus nicht ferromagnetischem Werkstoff, z.B. aus Aluminium. Der Hallgenerator 17 ist plättchenförmig ausgebildet und mit seiner wirksamen Fläche in einem Abstand d zum Antennenstab 18 so angeordnet, daß die Stabachse mit den Außenkanten des Plättchens in der Draufsicht (Fig. 3) ein gleichschenkeliges Dreieck bildet.

3

**0 004 888**

Die Verwendung von Mu-Metallstäben (im vorliegenden Ausführungsbeispiel etwa 1,5 mm stark) als Antennenstäbe und die geeignete Wahl des Abstandes d von etwa 3,5 mm führen zu einer linearisierten Charakteristik der Hallgeneratoren 17, wodurch sich die Spannungen an den Hallgeneratoren über die gesamte Länge der Mu-Metallstäbe nicht verändern. Aufgrund dieser Anordnung erhält man — wie nachfolgend noch näher erläutert-linearisierte Signale.

Wie in Fig. 2 schon angedeutet, verlaufen die von den Hallgeneratoren 17 zur Multiplexeinheit 11 (Fig. 1) führenden Leitungen 16 im Innern der Rohre 19, des Tragrahmens 6 und der Tragstange 7.

Die Fig. 4 zeigt in schematischer Darstellung nochmals die Anordnung der Magnetflußaufnehmer A bis F und die von einem Bezugspunkt P, der einer angenommenen Lage des Magnetfelderzeugers 3 in einer Ausgangsstellung entspricht, in den drei Ebenen des Raumes erhältlichen Signale, x, y und z für eine Translationsbewegung und $\alpha$, $\beta$ und $\gamma$ für eine Rotationsbewegung.

Aus der Anordnung wird für die Ausgangssignale (Gleichspannungssignale) an den Magnetflußaufnehmern bzw. Hallgeneratoren folgende Beziehung hergeleitet:

$$A = x + y + \alpha \qquad C = z + y + \gamma \qquad E = y + z + \beta$$

$$B = x - y - \alpha \qquad D = z - y - \gamma \qquad F = y - z - \beta$$

Aus der mathematischen Beziehung A + B ergibt sich, daß die Summe der Signale an den Aufnehmern A und B ein Maß für die reine Translationsbewegung in Richtung der x-Achse ist.

Aus der Formel

$$\text{I) } A + B = 2 \cdot x$$

kann also eine Translationsbewegung in Richtung der x-Achse gemessen werden.

Die Differenz der Signale A, B, also A—B, ergibt eine Mischung, von Translationsbewegung in y-Richtung und Rotationsbewegung um $\alpha$.

$$A - B = 2 (y + \alpha)$$

Für die Aufnehmer E und F gilt analog folgende Beziehung:

$$\text{II) } E + F = 2 \cdot y$$

Die Differenz der Signale E, F, also E—F, ergibt wiederum eine Mischung von Translationsbewegung in x-Richtung und Rotationsbewegung um $\beta$

$$E - F = 2 \cdot (x + \beta)$$

Aus der Formal

$$\text{III) } (A - B) - (E + F) = 2y + 2\alpha - 2y = 2\alpha$$

erhält man das Signal $\alpha$ als Maß für die Rotations um die y-Achse.

Entsprechendes ergibt sich für eine Bewegung in Richtung der z-Achse.

Aus der Formel

$$\text{IV) } C + D = 2 \cdot z$$

erhält man also die Signale z als Maß für die Translation in z-Richtung.

Die Differenz der Signale C—D ergibt wiederum eine Mischung von Translationsbewegung in y-Richtung und Rotationsbewegung um $\gamma$.

$$C - D = 2 \cdot (y + \gamma)$$

Aus den Formeln

$$\text{V) } (A + B) - (E - F) \qquad\qquad \text{und}$$

4

VI) (C − D) − (E + F)

erhält man die Signale $\beta$ und $\gamma$ als Maß für die Rotationsbewegungen um die y- und z-Achse.

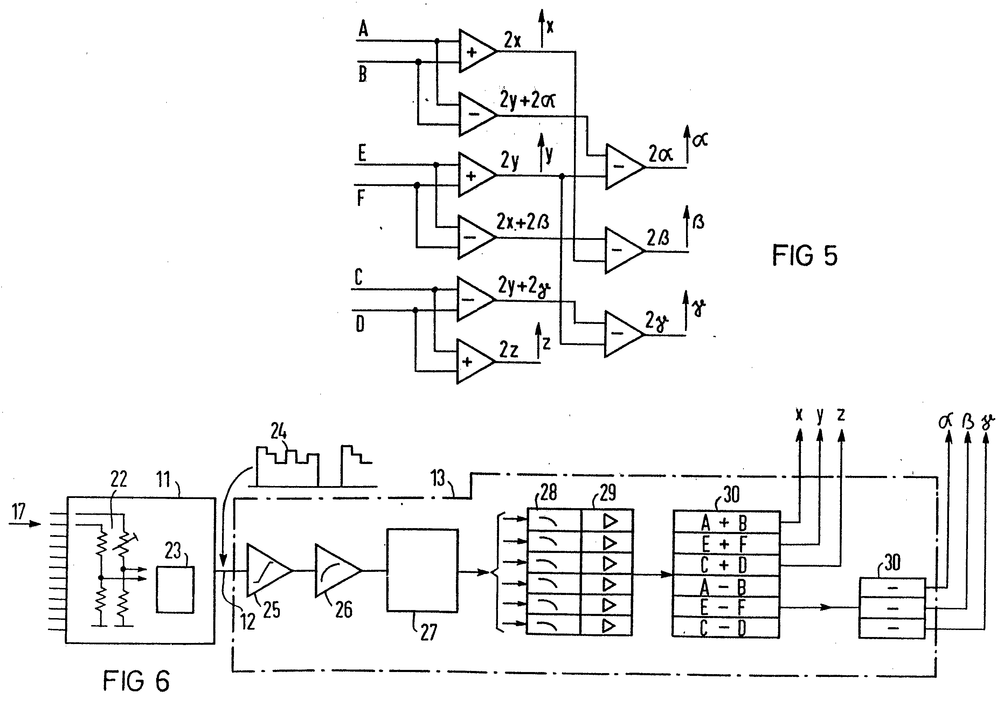

Werden als die Ausgangssignale entsprechend dem Blockschaltbild in Fig. 5 miteinander verknüpft, so gelingt es, die Rotations- und Translationssignale voneinander getrennt zu bekommen.

Die Fig. 6 zeigt anhand eines Blockschaltbildes die Signalverarbeitung ausgehend von der Multiplexeinheit 11, wobei von jedem Magnetflußaufnehmer A—F bzw. Hallgenerator 17 zwei Leitungen zur Multiplexeinheit 11 führen.

In der Multiplexeinheit 11 ist für jeden Hallgenerator 17 eine Brückenabgleichschaltung 22 vorhanden. Die Brückenabgleichschaltung 22 dient dazi, elektrische Unsymmetrien, die fertigungstechnisch bedingt sind, auszugleichen. Die von den sechs Hallgeneratoren gewonnenen Signale (Gleichspannungssignale) werden danach in einer Zeitmultiplexanordnung 23 in einer bestimmten Impulsfolge, die durch das Bild 24 angedeutet ist, codiert. Die Impulsfolge 24 wird dann über die Leitung 12 der Elektronikeinheit 13 zugeführt, und zwar zunächst einem Vorverstärker 25, dann einem Linearisierungsverstärker 26, einer Demultiplexschaltungsanordnung 27, anschliessend einem Filter 28 und einem weiteren Verstärker 29 und schließlich einem Rechner 30. Der Rechner 30 liefert letztlich analog der in Fig. 5 aufgezeigten Verknüpfung die einzelnen Signale für die Translationsund Rotationsbewegung.

## Patentansprüche

1. Vorrichtung zur Messung und Registrierung des Ortes, der Lage und/oder der Orts- bzw. Lageänderung eines starren Körpers im Raum, insbesondere einer Stelle des Unterkiefers eines Patienten, unter Verwendung eines am Körper direkt oder im Abstand davon angeordneten Magnetfelderzeugers, mit im Abstand vom Felderzeuger angeordneten, vom Körper unabhängigen Mitteln zur Erfassung des Feldflusses bzw. der Feldflußänderung während einer Messung sowie mit einer elektronischen Einrichtung zur Gewinnung und Auswertung von bei einem Feldfluß bzw. einer Feldflußänderung entstehenden elektrischen Signalen, dadurch gekennzeichnet, daß der Felderzeuger (3) so ausgebildet ist, daß er ein definiertes, in bezug auf seine Rotationssymmetrieachse unregelmäßiges Feld erzeugt, daß zur Erfassung des Feldflusses in wenigstens zwei, vorzugsweise in drei, senkrecht zueinander stehenden Ebenen in jeder Erfassungsebene eine Paar jeweils aus einem Hallgenerator (17) als Sensorelement und einem Antennenelement (18) gebildete Magnetflußaufnehmer (A/B; C/D; E/F) vorgesehen sind, die in einem Abstand (a, b, c) voneinander auf einem Träger (6) so angeordnet sind, daß zwischen den Magnetflußaufnehmern (A bis F) ein Freiraum gebildet ist.

2. Vorrichtung nach Anspruch 1, dadurch gekennzeichnet, daß die Antennenelemente jeweils einen in Richtung zum Magnetfelderzeuger (3) angeordneten, mit seiner Längsachse parallel zur Längssymmetrieachse des Hallgenerators (17) und in einem Abstand (d) dazu angeordneten Antennenstab (18) enthalten.

3. Vorrichtung nach Anspruch 2, dadurch gekennzeichnet, daß der Hallgenerator (17) auf halber Länge $(L_1)$ des Antennenstabes (18) derart angeordnet ist, daß in der Draufsicht (Fig. 3) die Außenkanten des Hallgenerators (17) mit dem Antennenstab (18) ein gleichschenkeliges Dreieck bilden.

4. Vorrichtung nach Anspruch 3, dadurch gekennzeichnet,daß als Antennenstab (18) ein Mu-Metallstab verwendet ist.

5. Vorrichtung nach einem der Ansprüche 2 bis 4, dadurch gekennzeichnet, daß der Hallgenerator (17) und der Antennenstab (18) in einem rohrförmigen Träger (19) aus einem nicht ferromagnetischen Material, vorzugsweise aus Aluminium, fixiert sind.

6. Vorrichtung nach Anspruch 5, dadurch gekennzeichnet, daß der Träger (19) mit einem weichen, formelastischen Mantel (21) umgeben ist.

7. Vorrichtung nach einem der Ansprüche 1 bis 6, dadurch gekennzeichnet, daß die Magnetflußaufnehmer (A bis F) an einem ⌐ -förmig ausgebildeten und mit seinen freien Schenkeln rechtwinkelig zueinander stehenden und wenigstens teilweise hohlen Tragrahmen (6) befestigt sind, und in den Hohlräumen die elektrischen Leitungen (16) von und zu den Hallgeneratoren (17) angeordnet sind.

8. Vorrichtung nach einem der Ansprüche 1 bis 7, dadurch gekennzeichnet, daß als Magnetfelderzeuger (3) zwei gleichartige Magnetfelderzeuger, vorzugsweise zwei Stabmagnete, verwendet sind, deren gleichnamige Pole (z.B. N—N) unter einem Winkel $(\varphi)$ kleiner als 180°, vorzugsweise zwischen 80 und 120°, aneinanderstoßen und daß die Magnetflußaufnehmer (A bis F) in bezug auf den Magnetfelderzeuger (3) so angeordnet sind, daß ein Aufnehmer (C) im wesentlichen vom Magnet fluß $(M_1)$, der von den aneinanderstoßenden Polen des Felderzeugers (3) ausgeht, und die übrigen Aufnehmer (A, B, D, E, F) vom Magnetfluß $(M_2)$, der von den freien Polen des Felderzeugers (3) ausgeht, beeinflußt werden.

9. Vorrichtung nach einem der Ansprüche 1 bis 8, dadurch gekennzeichnet, daß zur Signalverarbeitung die von den Hallgeneratoren (17) gewonnenen. Signale linearisiert und von jedem Aufnehmerpaar (A/B, C/D, E/F) Summen- und Differenzsignale gebildet werden, die durch mathe-

5

**0 004 888**

matische Verknüpfung miteinander getrennte Einzelsignale für die Translationsund Rotationsbewegung liefern.

10. Vorrichtung nach Anspruch 9, dadurch gekennzeichnet, daß die Signalverarbeitung nach der Beziehung

$$A + B = 2 \cdot x$$
$$E + F = 2 \cdot y$$
$$C + D = 2 \cdot z$$
$$(A - B) - (E + F) = 2 \cdot \alpha$$
$$(A + B) - (E - F) = 2 \cdot \beta$$
$$(C - D) - (E + F) = 2 \cdot \gamma$$

erfolgt, wobei x, y, z und $\alpha$, $\beta$, $\gamma$ die aus addition und/oder Subtraktion der Ausgangssignale an den Magnetflußaufnehmern (A – F) gewonnenen Signale als Maß für eine Translations- (x, y, z) bzw. Rotationsbewegung ($\alpha$, $\beta$, $\gamma$) darstellen.

11. Vorrichtung nach Anspruch 9, dadurch gekennzeichnet, daß eine Multiplexeinheit (11) mit einer Multiplexschaltungsanordnung (23) vorhanden ist, welche die von den Magnetflußaufnehmern (A – F) gewonnenen Signale in eine codierte Impulsfolge (24) unwandelt, und daß eine Demultiplexschaltungsanordnung (27) vorhanden ist, die die Impulsfolge (24) wieder in Einzelsignale decodiert.

12. Vorrichtung nach Anspruch 11, dadurch gekennzeichnet, daß die Multiplexeinheit (11) für jeden Magnetflußaufnehmer (A—F) eine Brückenabgleichschaltung (22) enthält.

13. Vorrichtung nach Anspruch 1, dadurch gekennzeichnet, daß die Magnetflußaufnehmer (A—F) an einen Tragrahmen (6) angeordnet sind, der an einem an einem Patienten anlegbaren Kopfgestell (8) befestigt ist.

14. Vorrichtung nach Anspruch 13, dadurch gekennzeichnet, daß das Kopfgestell (8) mehrere Gelenke (9) zur Anpassung an den Patientenkopf (1) enthält.

15. Vorrichtung nach Anspruch 9, dadurch gekennzeichnet, daß die Multiplexeinheit (11) für eine Halterung am Kopf (1) eines Patienten ausgebildet ist.

## Claims

1. A device for measuring and registering the location, posture and/or change of location or posture of a rigid body in space, in particular of a point at the lower jaw of a patient, employing a magnetic field source which is arranged directly at or in the vicinity of the body, field sensor means independent of the body to detect the field flux and change of field flux during a measuring process, and an electronic device for sensing and analysing electric signals produced by the magnetic field flux or change therein, characterised in that the field source (3) is designed to produce a defined field that is irregular with respect to its rotational axis of symmetry, and a respective pair of field sensors are provided to detect the field flux in at least two and preferably three detection planes mutually at right angles, each field sensor having a Hall generator (17) together with an antenna element (18), and being arranged on a carrier (6) at such a distance (a, b, c) from one another that a free space is established between the magnetic flux field sensors (A to F).

2. A device as claimed in Claim 1, characterised in that each antenna element comprises an antenna rod (18) arranged relative to the magnetic field source (3) with its longitudinal axis parallel to the longitudinal axis of symmetry of the Hall generator (17) and at a distance (d) therefrom.

3. A device as claimed in Claim 2, characterised in that the Hall generator (17) is arranged half way along $(L_1)$ the antenna rod (18) in such manner that in the top view (Figure 3) the outer edges of the Hall generator (17) together with the antenna rod (18) define an isosceles triangle.

4. A device as claimed in Claim 3, characterised in that the antenna rod (18) is of a Mu metal.

5. A device as claimed in any one of Claims 2 to 4, characterised in that the Hall generator (17) and the antenna rod (18) are fixed to a tube-shaped carrier (19) which consists of a non-ferromagnetic material, preferably of aluminium.

6. A device as claimed in Claim 5, characterised in that the carrier (19) is surrounded by a soft, deformable covering (21).

7. A device as claimed in any one of Claims 1 to 6, characterised in that the magnetic flux field sensors (A to F) are secured to a supporting frame (6) which is ⌐.-shaped and whose free sides are at right angles to one another, the frame having hollow spaces in which there are arranged the electric connection lines (16) for the Hall generators (17).

8. A device as claimed in any one of the Claims 1 to 7, characterised in that said magnetic source (3) comprises two magnetic field producers of the same type, preferably two bar magnets, whose like poles (e.g. N—N) abut an angle ($\varphi$) smaller than 180°, preferably between 80 and 120°, and that in relation to the magnetic field source (3) the magnetic flux field sensors (A to F) are arranged in such manner that one receiver (C) is predominantly affected by the magnetic flux $(M_1)$ which emanates from the abutting poles of the field source (3) and the remaining field sensors (A, B, D, E, F) are affected by the magnetic flux $(M_2)$ which emanates from the free poles of the field source (3).

6

9. A device as claimed in any one of Claims 1 to 8, characterised in that for signal processing the signals from the Hall generators (17) are linearized and used to produce summation and difference signals which supply separate individual signals for the motion of translation and motion of rotation by mathematical combination with one another by each receiver pair (A/B, C/D, E/F).

10. A device as claimed in Claim 9, characterised in that the signal processing occurs in accordance with the mathematical relations:—

$$A + B = 2 \cdot x$$
$$E + F = 2 \cdot y$$
$$C + D = 2 \cdot z$$
$$(A - B) - (E + F) = 2 \cdot \alpha$$
$$(A + B) - (E - F) = 2 \cdot \beta$$
$$(C - D) - (E + F) = 2 \cdot \gamma$$

where x, y, z and $\alpha$, $\beta$, $\gamma$ represent the signals produced by addition and/or subtraction of the output signals at the magnetic flux sensors (A — F) as a measure for a motion of translation (x, y, z) and for a motion of rotation $(\alpha, \beta, \gamma)$.

11. A device as claimed in Claim 9, characterised in that a multiplex unit (11) is provided having a multiplex circuit arrangement (23) which converts the signal from the magnetic flux sensors (A — F) into a coded pulse train (24), and a demultiplex circuit arrangement (27) is provided to decode the pulse train (24) into individual signals.

12. A device as claimed in Claim 11, characterised in that the multiplex unit (11) contains a bridge balance circuit (22) for each magnetic flux receiver (A—F).

13. A device as claimed in Claim 1, characterised in that the magnetic flux field sensors (A — F) are arranged on a supporting frame (6) secured to a head support (8) that can be affixed to a patient.

14. A device as claimed in Claim 13, characterised in that the head support (8) contains a plurality of joints (9) for adaptation to the patient's head (1).

15. A device as claimed in Claim 9, characterised in that the multiplex unit (11) is designed for a mounting support at the head (1) of a patient.

## Revendications

1. Dispositif pour mesurer et enregistrer l'emplacement, la position et/ou la variation d'emplacement ou de position d'un corps rigide dans l'espace, plus particulièrement d'une partie de la mâchoire inférieure d'un patient, moyennant l'utilisation d'un générateur de champ, de préférence d'un générateur de champ magnétique, disposé directement contre le corps ou à une certaine distance de ce dernier, et comportant des dispositifs prévus à une certaine distance du générateur de champ et indépendants du corps et permettant de détecter le flux du champ ou la variation du flux du champ pendant une mesure, ainsi qu'un dispositif électronique permettant d'obtenir et d'exploiter des signaux électriques appraissant pour un flux de champ ou pour une variation du flux de champ, caractérisé par le fait que le générateur de champ (3) est réalisé de telle manière qu'il produit un champ défini et irrégulier par rapport à son axe de symétrie de rotation, que pour la saisie du flux du champ dans au moins deux, de préférence trois plans perpendiculaires entre eux on prévoit dans chaque plan de saisie une paire de récepteurs du flux magnétique (A/B, C/D, E/F) dont chacun est formé par un générateur de Hall (17) formant élément de détection et par un élément d'antenne (18), lesdits récepteurs du flux magnétique étant disposés à des distances l'un de l'autre (a, b, c) sur un support (6) de manière qu'entre eux soit formé un espace libre.

2. Dispositif selon la revendication 1, caractérisé par le fait que chaque élément d'antenne comporte une tige d'antenne (18) disposée en direction du générateur de champ magnétique (3) avec son axe longitudinal parallèlement à l'axe longitudinal de symétrie du générateur de Hall (17) et à une distance (d) de ce dernier.

3. Dispositif selon la revendication 2, caractérisé par le fait que le générateur de Hall (17) est disposé au niveau de la moitié de la longueur $(L_1)$ de la tige d'antenne (18) de manière que, vues en plan (figure 3), les arêtes extérieures du générateur de Hall (17) forment, avec la tige d'antenne (18), un triangle isocèle.

4. Dispositif selon la revendication 3, caractérisé par le fait que l'on utilise comme tige d'antenne (18) une tige en mumétal.

5. Dispositif selon l'une des revendications 2 à 4, caractérisé par le fait que le générateur de Hall (17) et la tige d'antenne (18) sont fixés dans un support tubulaire (19) fait avec un matériau non ferromagnétique, de préférence en aluminium.

6. Dispositif selon la revendication 5, caractérisé par le fait que le support (19) est entouré d'une enveloppe molle et élastique (21).

7. Dispositif selon l'une des revendications 1 à 6, caractérisé par le fait que les récepteurs de flux magnétique (A à F) sont fixés à un cadre-support (6) au moins partiellement creux, réalisé sous la forme d'un ⌐ et dont les branches libres sont perpendiculaires entre elles, les conducteurs électriques (16) provenant des générateurs de Hall (17) ou menant à ces derniers, étant disposés dans les espaces creux.

8. Dispositif selon l'une des revendications 1 à 7, caractérisé par le fait que l'on utilise, comme générateur de flux magnétique (3), deux générateurs de champ magnétique d'un même genre, de préférence deux barreaux aimantés dont les pôles de même nom (par exemple N—N) se touchent sous un angle ($\varphi$) inférieur à 180°, de préférence compris entre 80 et 120°, et que les détecteurs de flux magnétique (A à F) sont disposés, de telle manière par rapport au générateur de flux magnétique, qu'un détecteur (C) est influencé essentiellement par le flux magnétique ($M_1$) émanant des pôles qui se contactent du générateur de champ (3), alors que les autres détecteurs (A, B, D, E, F) sont influencés par le flux magnétique ($M_2$) qui émane des pôles libres du générateur de champ (3).

9. Dispositif selon l'une des revendications 1 à 8, caractérisé par le fait que pour le traitement des signaux, les signaux obtenus par les générateurs de Hall (17) sont linéarisés et qu'on forme, de chaque paire de récepteurs (A/B, C/D, E/F) des signaux de sommation et de différences qui fournissent par une opération mathématique des signaux individuels séparés entre eux pour le mouvement de translation et pour le mouvement de rotation.

10. Dispositif selon la revendication 9, caractérisé par le fait que le traitement des signaux est opéré selon relations suivantes:

$$A + B = 2 \cdot x$$
$$E + F = 2 \cdot y$$
$$C + D = 2 \cdot y$$
$$(A - B) - (E + F) = 2 \cdot \alpha$$
$$(A + B) - (E - F) = 2 \cdot \beta$$
$$(C - D) - (E + F) = 2 \cdot \gamma$$

relations dans lesquelles x, y, z et $\alpha$, $\beta$, $\gamma$ représentent des signaux obtenus par addition et/ou soustraction des signaux de sortie au niveau des récepteurs de flux magnétique (A — F), signaux qui représentent la mesure pour un mouvement de translation (x, y, z) ou un mouvement de rotation ($\alpha$, $\beta$, $\gamma$).

11. Dispositif selon la revendication 9, caractérisé par le fait que l'on prévoit une unité de multiplexage (11) avec un montage multiplex (23), transformant les signaux obtenus par les récepteurs de flux magnétique (A — F) en un train d'impulsions (24), et que l'on prévoit un circuit de démultiplexage (27) qui décode le train d'impulsions (24) à nouveau en signaux individuels.

12. Dispositif selon la revendication 11, caractérisé par le fait que l'unité de multiplexage comporte pour chaque récepteur de flux magnétique (A — F) un circuit d'équilibrage en pont (22).

13. Dispositif selon la revendication 1, caractérisé par le fait que les récepteurs de flux magnétique (A — F) sont disposés sur un châssis de support (6) qui est fixé sur un appuie-tête (8) susceptible d'être appliqué contre le patient.

14. Dispositif selon la revendication 13, caractérisé par le fait quie l'appuie-tête (8) comprend plusieurs articulations (9) pour son adaptation à la tête (1) du patient.

15. Dispositif selon la revendication 9, caractérisé par le fait que l'unité de multiplexage (1) est réalisée de manière à pouvoir être maintenue sur la tête (1) d'un patient.

FIG 1

FIG 2

FIG 3

FIG 4

2

FIG 5

FIG 6

0 004 888